# EUROPÄISCHE PATENTSCHRIFT

(11) **EP 2 578 315 B1**
(45) Veröffentlichungstag und Bekanntmachung des Hinweises auf die Patenterteilung: **17.01.2018**
(21) Anmeldenummer: 12006260.9
(22) Anmeldetag: 05.09.2012
(51) Int. Cl.: C12M 1/36, B01L 1/02

(54) **Laboratoriumsbrutschrank mit verbesserter Innenraum-Befeuchtung**
Laboratory incubator with improved interior moistening
Incubateur de laboratoire avec humidification améliorée de l'espace intérieur

(30) Priorität: 05.10.2011 DE 102011114900
(43) Veröffentlichungstag der Anmeldung: 10.04.2013
(73) Patentinhaber: Thermo Electron LED GmbH, 63505 Langenselbold (DE)
(72) Erfinder: Pieczarek, Waldemar, 63505 Langenselbold (DE); Stahl, Hermann, 61130 Nidderau-Ostheim (DE)
(74) Vertreter: Tomerius, Isabel

(56) Entgegenhaltungen:
- EP-A1- 0 992 247
- DE-C1- 4 130 233
- US-A- 4 701 415

## Beschreibung

Die Erfindung betrifft einen Laboratoriumsbrutschrank, auch als Inkubator bezeichnet, insbesondere einen Begasungsbrutschrank, mit einem von einem Gehäuse umgebenden Innenraum und einem außerhalb des Innenraums angeordneten Dampferzeuger, der mit dem Innenraum über eine Dampfzuleitung in Verbindung steht.

Brutschränke dienen in Laboratorien üblicherweise dazu, in ihrem Innenraum Proben, insbesondere biologische und/oder mikrobiologische Proben, unter vorgegebenen Bedingungen wie einer bestimmten Temperatur und Luftfeuchtigkeit und - im Falle von Begasungsbrutschränken - einer definierten Gasatmosphäre zu lagern. Meist wird dabei versucht, die Bedingungen des menschlichen oder tierischen Körpers zu imitieren. Oft gewählte Bedingungen sind daher eine Temperatur von ca. 37 °C und eine möglichst hohe Luftfeuchtigkeit, die in der Regel bei mindestens 60 %, bevorzugt bei mindestens 80 %, besonders bevorzugt bei mindestens 90 % liegen sollte, ohne dass dabei jedoch an den Wänden oder anderen Bereichen des Brutschrankes Feuchtigkeit auskondensiert.

Im Stand der Technik sind verschiedene Möglichkeiten bekannt, in einem Laboratoriumsbrutschrank eine feuchte Innenraumatmosphäre zu erzeugen. Eine erste Möglichkeit besteht darin, im Inneren des Brutschrankes ein Wasserreservoir vorzusehen, aus dem durch Beheizen Wasser verdampft wird (zum Beispiel EP 1552888 A2). Die US 4,701,415 beschreibt ein System, bei dem die Luft aus dem Inneren des Brutschranks heraus, durch einen Befeuchter und wieder in das Innere des Brutschrankes hinein befördert wird. Im Befeuchter wird die Luft zur Anreicherung mit Wasser durch einen stehenden Wasserkörper geleitet. Der Wasserstand wird überwacht und bei zu geringem Pegel wird Wasser aus einem Behälter nachgefüllt, der sich über dem Befeuchter befindet. Das Wasser fließt hierbei allein durch die Schwerkraft in den Befeuchter nach, so lange ein Ventil geöffnet wird. Ein großes Problem dieser beiden Lösungen ist jedoch die leichte Verkeimung des Wasserbades und die Gefahr der Kontamination der im Brutschrank gelagerten Proben. Diese Verkeimungsgefahr kann deutlich reduziert werden, wenn dem Innenraum des Brutschranks von außerhalb überhitzter Wasserdampf zugeführt wird. Bekannt sind zum Beispiel Lösungen, in denen außerhalb des Brutschranks ein Dampfkessel aufgestellt ist, aus dem überhitzter Wasserdampf unter Druck in den Brutschrank zugeführt wird. Diese Lösung ist jedoch aufwendig und teuer, und die Regulierung der Dampfzufuhr schwierig. Zudem unterliegen diese Geräte den Sicherheitsanforderungen für Druckbehälter, in Deutschland beispielsweise der Betriebssicherheitsverordnung.

Es bestand also ein Bedarf an einem Laboratoriumsbrutschrank, in dessen Innenraum mit geringem Aufwand und ohne die Gefahr einer Dampfexplosion mit guter Regelbarkeit eine hohe Feuchtigkeit sichergestellt werden kann und die Gefahr der Kontamination möglichst gering gehalten wird. Aufgabe der Erfindung ist es, einen solchen Laboratoriumsbrutschrank anzugeben.

Die Lösung dieser Aufgabe gelingt mit dem Laboratoriumsbrutschrank gemäß Anspruch 1.

Bevorzugte Weiterbildungen des Laboratoriumsbrutschranks sind in den zugehörigen Unteransprüchen beschrieben.

Im Unterschied zum Stand der Technik, in dem Wasserdampf im Inneren des Laboratoriumsbrutschrankes erzeugt wird, ist in der vorliegenden Erfindung ein externer Dampferzeuger vorhanden. Damit wird die Anwesenheit eines verkeimenden Wasserbades im Inneren des Brutschrankes vermieden. Von den externen Dampferzeugern des Standes der Technik, die dem Innenraum des Brutschrankes überhitzten Wasserdampf unter Druck zuführen, unterscheidet sich die Erfindung dadurch, dass der über die Wasserzuleitung mit dem Dampferzeuger verbundene Wasserbehälter wie ein Überdruckventil für den Dampferzeuger fungiert. Die Gefahr von Dampfexplosionen entfällt somit, ohne dass es zu Beschränkungen bei der kontinuierlichen Bereitstellung von Wasserdampf kommt. Der Dampferzeuger unterfällt auch nicht den einschlägigen Vorschriften für Druckbehälter oder Dampfkessel wie beispielsweise der Betriebssicherheitsverordnung oder deren Vorgängern Druckbehälter- und Dampfkesselverordnung. Dies erleichtert Herstellung, Zulassung und Wartung im Vergleich zu herkömmlichen Dampfdruckbehältern erheblich.

Dennoch erlaubt es die erfindungsgemäße Konstruktion, im Dampferzeuger einen Überdruck zu erzeugen, der zudem durch entsprechende Anordnung des Wasserbehälters auf sehr einfache Weise einstellen und gleichmäßig regeln lässt. Der maximale Druck, der im Dampferzeuger herrscht, ergibt sich aus der Höhe der Wassersäule, die in Wasserbehälter und Wasserzuleitung, bei der es sich zweckmäßig um eine offene Leitung ohne Absperrhähne oder Ventile handelt, oberhalb des Wasserspiegels des Dampferzeugers steht. Der Wasserbehälter wird also zweckmäßig so relativ zum Dampferzeuger angeordnet, dass sich während der Dampferzeugung aufgrund der Differenz der Wasserspiegel ein gewünschter Überdruck im Dampferzeuger einstellt. Für eine unter den Betriebsbedingungen ausreichende Wassersäule und damit einen ausreichenden Überdruck kann durch entsprechende Dimensionierung, Anordnung und Befüllung des Wasserbehälters gesorgt werden. Bevorzugt wird der Wasserbehälter oberhalb des Dampferzeugers angeordnet, wobei es ausreicht, wenn die Höhe der Böden, auf denen das Wasserreservoir jeweils steht, unterschiedlich hoch ist. Ein ausreichender Druck im Dampferzeuger wird in der Regel erzielt, wenn während der Dampferzeugung die Höhendifferenz der Wasserspiegel in Wasserbehälter und Dampferzeuger bei mindestens 6 cm und bevorzugt zwischen 6 und 60 cm liegt. Besonders bevorzugt beträgt die Höhendifferenz 15 bis 60 cm, und insbesondere liegt sie zwischen 20 und 30 cm.

Wird das Wasserreservoir im Dampferzeuger mittels der Heizvorrichtung, beispielsweise einem Plattenheizkörper im Bodenbereich des Dampferzeugers, erhitzt, vorzugsweise bis zum Sieden, baut sich im Inneren des Dampferzeugers ein Überdruck auf. Hierzu ist zweckmäßig ein Ventil in der Dampfzuleitung zum Brutschrank hin vorgesehen, das nur bei Bedarf geöffnet wird, um Dampf in den Innenraum des Brutschranks hineinzulassen. Die Regulierung der Dampfzufuhr geschieht in an sich bekannter Weise aufgrund der im Innenraum ermittelten Feuchtigkeits- und Temperaturwerte. Sinken die Werte unter einen vorgegebenen Sollwert, wird das Ventil geöffnet und Dampf strömt in den Innenraum ein, bis die vorgegebenen Werte erreicht sind. Auch die Heizvorrichtung des Dampferzeugers kann in üblicher Weise, beispielsweise von der Steuerung des Brutschranks, geregelt werden. Bevorzugt wird zur Einstellung der Temperatur des Wasserreservoirs ein PTC-Element (PTC = positive temperature coefficient) eingesetzt. Dieses erhöht bei steigender Temperatur seinen Widerstand und stellt sich so selbstregelnd auf die gewünschte Temperatur ein. Alternativ ist aber zum Beispiel auch eine elektronische Temperatursteuerung möglich. Zweckmäßig wird die Heizvorrichtung des Dampferzeugers kontinuierlich betrieben, um permanent während des Betriebs Wasserdampf zur Verfügung zu stellen. Theoretisch ist aber auch ein nur phasenweiser Betrieb der Heizvorrichtung denkbar.

Steigt der Druck im Dampferzeuger an, wird die Wassersäule, die in der Wasserzuleitung und im Wasserbehälter steht, in Richtung auf den Wasserbehälter geschoben. Zweckmäßig ist hier, wenn die Wasserzuleitung den Wasserbehälter in einem Bodenbereich verlässt und in einen Bodenbereich des Dampferzeugers einmündet. Weiter trifft das im Dampferzeuger erhitzte Wasser in der Wasserzuleitung auf kälteres Wasser, kühlt sich dadurch ab, und eventuell eintretender Dampf kondensiert aus. Aufgrund der so erfolgenden Temperaturreduzierung nimmt der Druck im Dampferzeuger ab. Bei entsprechender Auslegung und Anordnung des Wasserbehälters ist die Höhendifferenz der Wasserspiegel in Dampferzeuger und Wasserbehälter groß genug, dass das in der Wasserzuleitung stehende Wasser durch den Dampf nicht völlig aus der Leitung verdrängt wird. Sollte dies bei maximalem Druck doch einmal passieren, kann der Dampf gefahrlos durch den Wasserbehälter entweichen, da der Wasserbehälter druckoffen ausgelegt ist. Zu diesem Zweck kann beispielsweise eine Druckausgleichsöffnung in der Behälterwandung vorgesehen sein, die bevorzugt mit einem (Steril)filter versehen ist. Grundsätzlich könnte der Behälter auch nach oben zumindest teilweise offen sein. Dies ist jedoch zur Vermeidung von Verkeimung und aus energetischer Sicht nicht bevorzugt. Zweckmäßig kann der Behälter vielmehr mit einer Isolierung versehen sein, um einen Wärmeabfluss zu vermeiden.

Zur Verhinderung einer Kontamination des Wasserreservoirs ist es zudem bevorzugt, den Wasserbehälter ebenfalls mit einer Heizvorrichtung auszustatten, um damit das Wasserreservoir beheizen zu können. Zweckmäßig ist eine zumindest zeitweilige Erwärmung auf eine Temperatur zwischen 60 °C und unter 100 °C. Unter 60 °C wird keine Wirkung im Hinblick auf eine Kontaminationsvermeidung oder -beseitigung erzielt. Um ein Sieden des Wasserreservoirs im Wasserbehälter zu vermeiden, wird die Temperatur zudem zweckmäßig unter 100 °C gehalten. Besonders bevorzugt wird die Temperatur auf 70 bis 90 °C und insbesondere auf ca. 80 °C (± 5 °C) eingestellt. Die Erwärmung des Wasserreservoirs im Wasserbehälter hat zudem den Vorteil, dass die Temperaturerhöhung des Wassers im Dampferzeuger bis zum Sieden geringer ist als bei nicht vorgewärmtem Wasser. Der Dampferzeuger kann deshalb kleiner und mit geringerer Leistung ausgelegt werden. Die Temperatureinstellung erfolgt wie im Dampferzeuger, also bevorzugt mittels PTC-Element.

Die Einleitung des im Dampferzeuger erzeugten Wasserdampfes in den Innenraum des Brutschrankes erfolgt über eine Dampfzuleitung, die bevorzugt vom Dampferzeuger zur Einmündung in den Innenraum des Brutschrankes hin ansteigt. Der Vorteil dieser Anordnung besteht darin, dass in der Dampfzuleitung auskondensierter Dampf in Richtung auf den Dampferzeuger zurückläuft und so nicht in den Innenraum spritzt und dort vorhandene Proben verunreinigt. Besonders bevorzugt ist es, wenn eine Rücklaufleitung von der Dampfzuleitung in den Wasserbehälter geführt ist. Dann läuft in der Dampfzuleitung auskondensiertes und abgekühltes Wasser nicht in den Dampferzeuger zurück, sondern in den geringer temperierten Wasserbehälter. Wird die Rücklaufleitung zwischen dem Ventil und der Einmündung in den Innenraum aus der Dampfzuleitung herausgeführt, wird vorteilhaft verhindert, dass Wasser vor dem Ventil stehen bleibt und von dort mit dem nächsten Dampfeinlass in den Innenraum mitgeschleppt und dort verspritzt wird. Um ein Auskondensieren von Wasser in der Dampfzuleitung möglichst zu verhindern, kann diese, insbesondere im Bereich zwischen Ventil und Brutschrank, zusätzlich isoliert und/oder beheizt werden.

Im Vorstehenden wurde die Erfindung so beschrieben, dass für einen Brutschrank ein Dampferzeuger vorgesehen ist. Es ist jedoch ebenfalls möglich, mehrere Brutschränke mit einem Dampferzeuger zu verbinden. Zu diesem Zweck führt aus dem Dampferzeuger entweder mehr als eine Dampfzuleitung heraus, nämlich pro Brutschrank jeweils eine Dampfleitung, oder aber eine aus dem Dampferzeuger herausführende Dampfzuleitung wird auf mehrere Brutschränke aufgeteilt. Zweckmäßig ist dann in jeder der Dampfleitungen ein Ventil vorhanden, das gesondert angesteuert werden kann, so dass die Dampfzufuhr zu jedem der Brutschränke individuell eingestellt werden kann. Die Verwendung nur eines Dampferzeugers für mehrere Brutschränke senkt den Platzbedarf und die Kosten für die Gesamtanordnung.

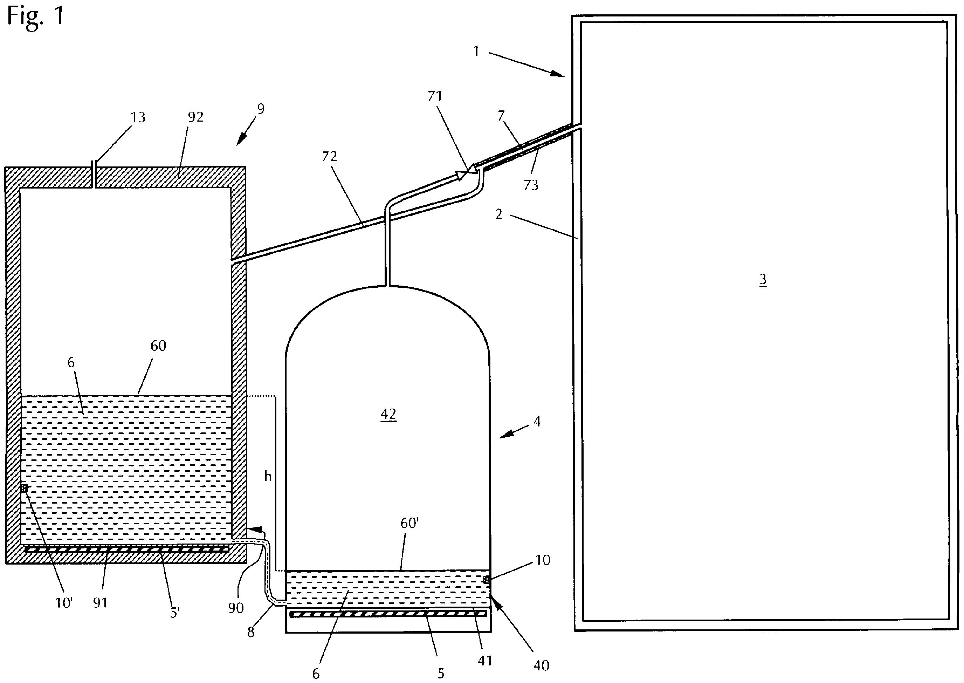

Die Erfindung soll nachfolgend anhand einer Zeichnung näher erläutert werden. In der schematischen und nicht maßstabsgerechten Zeichnung zeigt
- Fig. 1: einen Querschnitt durch einen erfindungsgemäßen Laboratoriumsbrutschrank mit angeschlossenem Dampferzeuger.

Figur 1 beschreibt die Erfindung am Beispiel eines Brutschrankes, insbesondere Inkubators, 1. Der Inkubator 1 umfasst ein Gehäuse 2, das einen Innenraum 3 umschließt, in dem Proben wie zum Beispiel biologische oder mikrobiologische Proben unter vorgegebenen Temperatur-, Feuchtigkeits- und Gasatmosphären-Bedingungen gelagert werden können. Übliche Lagerbedingungen für die Proben sind beispielsweise eine Temperatur von 37 °C und eine Luftfeuchtigkeit von etwa 95 %. Der Innenraum 3 ist durch eine im Querschnitt nicht erkennbare Tür verschließbar. Die Innenausstattung wie beispielsweise Tragböden, Messeinrichtungen usw. ist zur Vereinfachung weggelassen.

Zur Erzeugung der im Innenraum 3 benötigten Feuchtigkeit ist mit dem Inkubator 1 ein Dampferzeuger 4 über eine Dampfzuleitung 7 verbunden. Der Dampferzeuger 4 besteht aus einem Behälter mit einem Bodenbereich 40, der ein Wasserreservoir 6 aufnehmen kann, und einem darüber liegenden, hier kuppelförmig ausgebildeten Dampfraum 42. Im Dampferzeuger 4 wird Wasserdampf durch Erhitzen des Wasserreservoirs 6 mittels einer Heizvorrichtung 5 erzeugt und aus dem Dampfraum 42 über die Dampfzuleitung 7, die den Dampferzeuger am höchsten Punkt des Dampfraumes 42 verlässt, dem Innenraum 3 des Inkubators 1 zugeleitet. Die Dampfzuleitung 7 ist mit einem Ventil 71 verschlossen, das in an sich bekannter Weise immer dann geöffnet wird, wenn die im Innenraum 3 gemessenen Feuchtigkeits- und Temperaturwerte unter vorgegebene Sollwerte fallen. Nach Erreichen der Sollwerte wird das Ventil 71 wieder geschlossen und die Dampfzufuhr unterbrochen.

Der Dampferzeuger 4 ist über eine aus seinem Bodenbereich 40 herausführende Wasserzuleitung 8 in Form eines unverschlossenen Rohres mit einem Wasserbehälter 9 verbunden und so angeordnet, dass der Boden 41 des Dampferzeugers 4 unterhalb des Bodens 91 des Wasserbehälters 9 liegt. Die Wasserzuleitung 8 führt aus einem Bodenbereich 90 des Wasserbehälters 9 heraus. Das Wasserreservoir 6 verteilt sich so auf den Dampferzeuger 4 und den Wasserbehälter 9. Wird das Wasserreservoir 6 im Dampferzeuger durch Betreiben der Heizvorrichtung 5 auf bevorzugt ca. 100 °C erhitzt, bildet sich Wasserdampf und füllt den Dampfraum 42 an. Dadurch steigt der Druck im Dampferzeuger 4, der Wasserspiegel 60' sinkt und Wasser wird aus dem Dampferzeuger 4 über die Wasserzuleitung 8 in den Wasserbehälter 9 verdrängt. Dort steigt der Wasserspiegel 60 an. Fig. 1 zeigt dies, also einen Zustand während der Erzeugung von Wasserdampf im Dampferzeuger 4. Der Dampfdruck im Dampferzeuger 4 entspricht der Wassersäule, die oberhalb des Wasserspiegels 60' steht, also der Differenz der Wasserspiegel 60 und 60' (Höhe h).

Die Anordnung des Wasserbehälters 9 relativ zum Dampferzeuger ist derart, dass unter normalen Umständen das in der Wasserzuleitung 8 stehende Wasser nicht vollständig aus der Leitung verdrängt wird. Eventuell in die Wasserzuleitung 8 eindringender Dampf kommt mit dem kälteren Wasser in der Leitung 8 in Kontakt, kühlt ab und kondensiert aus, wodurch der Druck absinkt. Zum Ausgleich sehr hohen Überdrucks ist im Wasserbehälter 9 zudem eine Druckausgleichsöffnung 13 vorhanden, durch die eventuell doch in den Wasserbehälter 9 eindringender Dampf entweichen kann. Dampfexplosionen werden auf diese Weise sicher vermieden.

Das Wasserreservoir 6 im Wasserbehälter 9 muss prinzipiell gar nicht erwärmt werden. Im gezeigten Fall ist jedoch eine Heizvorrichtung 5' vorhanden, mit der das Wasserreservoir 6 auch im Wasserbehälter 9 erwärmt wird, wenn auch auf eine niedrigere Temperatur als im Dampferzeuger 4. Vorzugsweise wird auf eine Temperatur von mindestens 60 °C, besonders bevorzugt auf 80 bis 90 °C erhitzt. Je höher die Temperatur des Wasserreservoirs 6, desto geringer die Wahrscheinlichkeit, dass sich Keime im Wasser bilden, und desto geringer die Heizleistung, um Wasser im Dampferzeuger 4 zum Sieden zu bringen. Die Temperatur des Wasserreservoirs 6 im Wasserbehälter 9 sollte jedoch nicht so hoch sein, dass das Wasser dort zu sieden beginnt. Die Überwachung der Temperatur erfolgt mit einem PTC-Element 10', das (hier nicht dargestellt) mit der Stromversorgung der Heizvorrichtung 5' verbunden ist. In analoger Weise erfolgt die Temperaturkontrolle im Dampferzeuger 4 mit einem PTC-Element 10. Um den Wärmeverlust zu vermindern, ist der Wasserbehälter 9 mit einer Isolierung 92 versehen.

Bei der Zufuhr von Wasserdampf unter Druck trat im Stand der Technik häufig das Problem auf, dass in der Dampfzuleitung auskondensiertes Wasser von einem Dampfstoß in den Innenraum des Brutschrankes mitgeschleppt und dort verspritzt wurde und sich dann auf den Proben niederschlug. Im erfindungsgemäßen Brutschrank wird dies dadurch vermieden, dass die Dampfzuleitung 7 schräg in Richtung auf den Innenraum 3 ansteigend angeordnet ist. So läuft Kondensationswasser nicht in den Innenraum 3, sondern in Richtung auf den Dampferzeuger 4 zurück. Damit es sich nicht vor dem Ventil 71 ansammelt, ist zudem eine Rücklaufleitung 72 vorgesehen, die zwischen Ventil 71 und Einlass in den Brutschrank nach unten aus der Dampfzuleitung 7 abzweigt und in den Wasserbehälter 9 zurückführt. Außerdem ist der Bereich der Dampfzuleitung 7 zwischen Ventil 71 und Brutschrank 1 mit einer Isolierung 73, bei der es sich auch um eine Heizmanschette handeln kann, versehen, um ein Auskondensieren von Wasser hier möglichst zu vermeiden.

## Patentansprüche

1. Laboratoriumsbrutschrank (1), insbesondere Begasungsbrutschrank, mit einem von einem Gehäuse (2) umgebenen Innenraum (3) und einem außerhalb des Innenraums (3) angeordneten Dampferzeuger (4), der eine Heizvorrichtung (5) zur Erwärmung eines Wasserreservoirs (6) und zur Erzeugung von Wasserdampf aufweist und mit dem Innenraum (3) über eine Dampfzuleitung (7) in Verbindung steht, wobei der Dampferzeuger (4) über eine Wasserzuleitung (8) mit einem druckoffenen Wasserbehälter (9) verbunden ist, und wobei der Wasserbehälter (9) derart angeordnet ist, dass der Wasserspiegel (60) im Wasserbehälter (9) während der Dampferzeugung oberhalb des Wasserspiegels (60') im Dampferzeuger (4) liegt,
**dadurch gekennzeichnet,**
**dass** die Wasserzuleitung (8) eine offene Leitung ohne Absperrhähne oder Ventile ist, so dass sich während der Dampferzeugung aufgrund der Differenz der Wasserspiegel (60, 60') ein gewünschter Überdruck im Dampferzeuger (4) einstellt.

2. Laboratoriumsbrutschrank nach Anspruch 1,
**dadurch gekennzeichnet,**
**dass** die Wasserzuleitung (8) den Wasserbehälter (9) in einem Bodenbereich (90) verlässt und in einen Bodenbereich (40) des Dampferzeugers (4) einmündet.

3. Laboratoriumsbrutschrank nach Anspruch 1 oder 2,
**dadurch gekennzeichnet,**
**dass** die Höhendifferenz (h) der Wasserspiegel (60, 60') bei mindestens 6 cm, bevorzugt zwischen 6 und 60 cm, besonders bevorzugt zwischen 15 cm und 60 cm und insbesondere zwischen 20 und 30 cm liegt.

4. Laboratoriumsbrutschrank nach einem der Ansprüche 1 bis 3,
**dadurch gekennzeichnet,**
**dass** die Dampfzuleitung (7) vom Dampferzeuger (4) zur Einmündung in den Innenraum (3) hin ansteigt.

5. Laboratoriumsbrutschrank nach einem der Ansprüche 1 bis 4,
**dadurch gekennzeichnet,**
**dass** die Dampfzuleitung (7) mit einem Ventil (71) versehen ist.

6. Laboratoriumsbrutschrank nach Anspruch 5,
**dadurch gekennzeichnet,**
**dass** eine Rücklaufleitung (72) von der Dampfzuleitung (7) in den Wasserbehälter (9) geführt ist, wobei die Rücklaufleitung (72) bevorzugt zwischen dem Ventil (71) und der Einmündung in den Innenraum (3) aus der Dampfzuleitung (7) führt.

7. Laboratoriumsbrutschrank nach einem der Ansprüche 5 oder 6,
**dadurch gekennzeichnet,**
**dass** die Dampfzuleitung (7), insbesondere im Bereich zwischen dem Ventil (71) und dem Laboratoriumsbrutschrank (1), mit einer Isolation (73) und/oder Heizvorrichtung versehen ist.

8. Laboratoriumsbrutschrank nach einem der Ansprüche 1 bis 7,
**dadurch gekennzeichnet,**
**dass** der Wasserbehälter (9) mit einer Heizvorrichtung (5') zur Erwärmung des Wasserreservoirs (6), bevorzugt auf eine Temperatur zwischen 60 °C und unter 100 °C, besonders bevorzugt auf 70 bis 90 °C und insbesondere auf ca. 80 °C aufweist.

9. Laboratoriumsbrutschrank nach einem der Ansprüche 1 bis 8,
**dadurch gekennzeichnet,**
**dass** zur Einstellung der Temperatur des Wasserreservoirs (6) im Dampferzeuger (4) und/oder im Wasserbad (9) ein PTC-Element (10, 10') vorhanden ist.

## Claims

1. Laboratory incubator (1), in particular a gassed incubator, with an interior (3) surrounded by a housing (2) and a steam generator (4) which is arranged outside the interior (3), which comprises a heating device (5) for heating a water reservoir (6) and for generating steam and which is connected with the interior (3) via a steam supply line (7), wherein the steam generator (4) is connected with an open-pressure water container (9) and wherein the water container (9) is arranged in such a manner that the water level (60) in the water container (9) during the generation of steam lies above the water level (60') in the steam generator (4), **characterized in that** the water supply line (8) is an open line without shut-off valves or other valves so that the desired positive pressure in the steam generator (4) is created during the steam generation as a result of the difference between the water levels (60, 60').

2. Laboratory incubator according to claim 1,
**characterized in that** the water supply line (8) leaves the water container (9) in a base region (90) and feeds into a base region (40) of the steam generator (4).

3. Laboratory incubator according to claims 1 or 2,
**characterized in that** the difference in height (h) between the water levels (60, 60') is at least 6 cm, preferably between 6 and 60 cm, particularly preferably between 15 cm and 60 cm and especially between 20 and 30 cm.

4. Laboratory incubator according to claims 1 to 3,
**characterized in that** the steam supply line (7) rises from the steam generator (4) to the opening into the interior (3).

5. Laboratory incubator according to claims 1 to 4,
**characterized in that** the steam supply line (7) comprises a valve (71).

6. Laboratory incubator according to claims 1 to 5,
**characterized in that** a return flow line (72) is guided from the steam supply line (7) into the water container (9), the return flow line (72) preferably leading out of the steam supply line (7) between the valve (71) and the opening into the interior (3).

7. Laboratory incubator according to claims 1 to 6,
**characterized in that** the steam supply line (7), in particular in the region between the valve (71) and the laboratory incubator (1), is provided with an insulation (73) and/or heating device.

8. Laboratory incubator according to claims 1 to 7,
**characterized in that** the water container (9) has a heating device (5') for heating the water reservoir (6), preferably to a temperature between 60 °C and below 100 °C, particularly preferably between 70 and 90 °C, and especially to approximately 80 °C.

9. Laboratory incubator according to claims 1 to 8,
**characterized in that** a PTC element (10, 10') is provided for setting the temperature of the water reservoir (6) in the steam generator (4) and/or in the water bath (9).

## Revendications

1. Incubateur de laboratoire (1), plus particulièrement incubateur à gaz, avec un intérieur (3) entouré par un boîtier (2) et un générateur de vapeur (4) qui est disposé en dehors de l'intérieur (3), qui comprend un dispositif de chauffage (5) pour chauffer un réservoir d'eau (6) et pour générer du vapeur d'eau et qui est relié à l'intérieur (3) via une conduite d'alimentation de vapeur (7), le générateur de vapeur (4) étant relié à un conteneur d'eau (9) à pression ouverte et le conteneur d'eau (9) étant disposé de telle sorte que le niveau d'eau (60) dans le conteneur d'eau (9) soit au-dessus du niveau d'eau (60') dans le générateur de vapeur (4) lors de la génération du vapeur,
**caractérisé en ce que** la conduite d'alimentation d'eau (8) est une conduite ouverte sans vannes de fermeture ou d'autres soupapes afin que la pression positive désirée dans le générateur de vapeur (4) soit créée lors de la génération du vapeur en conséquence de la différence entres les niveaux d'eau (60, 60').

2. Incubateur de laboratoire (1) selon la revendication 1,
**caractérisé en ce que** la conduite d'alimentation d'eau (8) quitte le conteneur d'eau (9) dans la région de base (90) et alimente une région de base (40) du générateur de vapeur (4).

3. Incubateur de laboratoire selon la revendication 1 ou 2,
**caractérisé en ce que** la différence en hauteur (h) des niveaux d'eau (60, 60') est au moins 6 cm, de préférence entre 6 et 60 cm, de manière plus préférée entre 15 cm et 60 cm et en particulier entre 20 et 30 cm.

4. Incubateur de laboratoire selon l'une quelconque des revendications 1 à 3,
**caractérisé en ce que** la conduite d'alimentation de vapeur (7) monte du générateur de vapeur (4) jusqu'à l'ouverture dans l'intérieur (3).

5. Incubateur de laboratoire selon l'une quelconque des revendications 1 à 4,
**caractérisé en ce que** la conduite d'alimentation de vapeur (7) est équipée d'une soupape (71).

6. Incubateur de laboratoire selon la revendication 5,
**caractérisé en ce qu'**une conduite à reflux (72) est guidée de la conduite d'alimentation de vapeur (7) au conteneur d'eau (9), la conduite à reflux (72) sortant de manière préférée de la conduite d'alimentation de vapeur (7) entre la valve et l'ouverture dans l'intérieur (3).

7. Incubateur de laboratoire selon la revendication 5 ou 6,
**caractérisé en ce que** la conduite d'alimentation de vapeur (7), en particulier dans la région entre la soupape (71) et l'incubateur de laboratoire (1), est pourvue d'une isolation (73) et/ou d'un dispositif de chauffage.

8. Incubateur de laboratoire selon l'une quelconque des revendications 1 à 7,
**caractérisé en ce que** le conteneur d'eau (9) a un dispositif de chauffage (5') pour chauffer le réservoir d'eau (6), de préférence à un température entre 60 °C et sous 100 °C, da manière plus préférée entre 70 et 90 °C, et en particulier à environ 80 °C.

9. Incubateur de laboratoire selon l'une quelconque des revendications 1 à 8,
**caractérisé en ce qu'**un élément CTP (10, 10') est présent pour le règlement de la température du réservoir d'eau (6) dans le générateur de vapeur (4) et/ou dans le bain d'eau (9).
